Europäisches Patentamt

European Patent Office

Office européen des brevets

(19)

(11) Publication number: **0 019 444**

**A1**

(12) **EUROPEAN PATENT APPLICATION**

(21) Application number: **80301547.8**

(22) Date of filing: **12.05.80**

(51) Int. Cl.³: **A 61 B 5/00**
**A 61 F 7/00, A 61 H 33/06**
**A 61 B 5/04**

(30) Priority: **16.05.79 GB 7917099**

(43) Date of publication of application:
**26.11.80 Bulletin 80/24**

(84) Designated Contracting States:
**AT BE CH DE FR IT LI NL**

(71) Applicant: **UNIVERSITY OF BRADFORD**

**Bradford, West Yorkshire, BD7 1DP(GB)**

(72) Inventor: **Harness, John Barrington**
**6, Woodlands Grove Baildon**
**Shipley, West Yorkshire, BD17 5BD(GB)**

(72) Inventor: **Mearns, Alan James**
**5, North Parade West Park**
**Leeds, West Yorkshire, LS16 5AY(GB)**

(74) Representative: **Geldard, David Guthrie et al,**
**Urquhart-Dykes & Lord 11th Floor Tower House Merrion**
**Way**
**Leeds, LS2 8PB(GB)**

(54) **Medical or veterinary equipment.**

(57) Apparatus for determining the response of an animal body to periodic application of an external stimulus includes means (23, 25) for applying alternating hot and cold gas to an appropriate part of the animal body, for instance, to the space within the sleeve (13) enclosing the limb of the animal's body. A periodic signal generated by the animal body is detected (27, 29) during said application and presented in a form suitable for assessing the response of the signal to the application of the alternating hot and cold air, thereby allowing information to be determined about the condition of the animal body.

EP 0 019 444 A1

./...

Croydon Printing Company Ltd.

*FIG.3*

1 — Air at constant flow and pressure

3

COOLER 7

5 — 11 — Variable electric supply

9

to timing mechanism — 23

25 — to timing mechanism

13 — LIMB SLEEVE

to atmosphere

- 1 -

## MEDICAL OR VETERINARY EQUIPMENT

This invention relates to medical or veterinary equipment and in particular to equipment for determining the response of an animal body to an applied stimulus.

It has previously been shown that entrainment of the thermoregulatory control system of the human body can take place. When used herein the term entrainment refers to the ability of a control system of an animal body to respond to an appropriate and periodically externally applied stimulus, for instance a thermal stimulus in the case of the thermoregulatory control system. The entrainment of the body temperature control mechanism has been shown by measuring the blood flow signal over a period during which a subject placed one hand alternately at regular intervals in two buckets of water which were at different temperatures.

According to the present invention there is provided apparatus for determining the response of an animal body to periodic application of an external stimulus, the apparatus comprising means for applying alternating hot and cold gas to at least a part of the animal body, means for presenting a periodic signal generated by the animal body, and detected during the application of said hot and cold gas, in a form suitable for assessing the response of said signal to said application of alternating hot and cold air.

The apparatus may include means for measuring said periodic signal.

Accordingly, apparatus in accordance with the present invention does not require any movement by the subject, whether a human or non-human animal subject.

Preferably the means for presenting the periodic signal include means for accurately recording the intervals between characteristic repeat portions of the signal and means for analysing the results obtained to isolate the contribution to the signal provided by the thermoregulatory control mechanism. Preferably the analysis is by means of Fourier transform techniques. By such techniques it is possible to obtain the required information regarding the efficiency of entrainment from the cardiac rate variability as from the peripheral blood flow signal.

Preferably the gas is air and it is applied to a limb of the animal, such as an arm or leg. The animal may be human or non-human, such as a horse.

The present invention also provides a method for determining the response of an animal body to periodic application of an external stimulus, the method comprising applying alternating hot and cold gas to at least a part of the animal body, detecting a periodic signal generated by the animal body during the application of said alternating hot and cold air, and presenting the detected periodic signal in a form suitable for assessment of the response of said signal to said application of alternating hot and cold air.

Appropriate periodic body signals may be obtained from one or more of the following:-

(1)    The  electrocardiogram (E.C.G.);

(2 )   The blood flow through the peripheral circulation measured by a plethysmograph usually on a finger or ear lobe;

(3)    Blood  pressure signals from either a transducer in a blood vessel or a plethysmograph;

(4)    The intercranial pressure (I.C.P.) measured by inserting a pressure transducer extra-durally or attached to a catheter in the ventricle;

(5)    Blood flow fron a continuous gated dopler device;

(6)    Respiration rate measured by either a thyristor in the nose or a strain gauge around the chest or abdomen.

All the above signals contain similar information, with the exception of the respiration rate signal which contains only part of the information derivable from the other signals, and the signals may be analysed by several techniques. Measurement of at least one signal, preferably two or three, should ensure that the information is obtained.

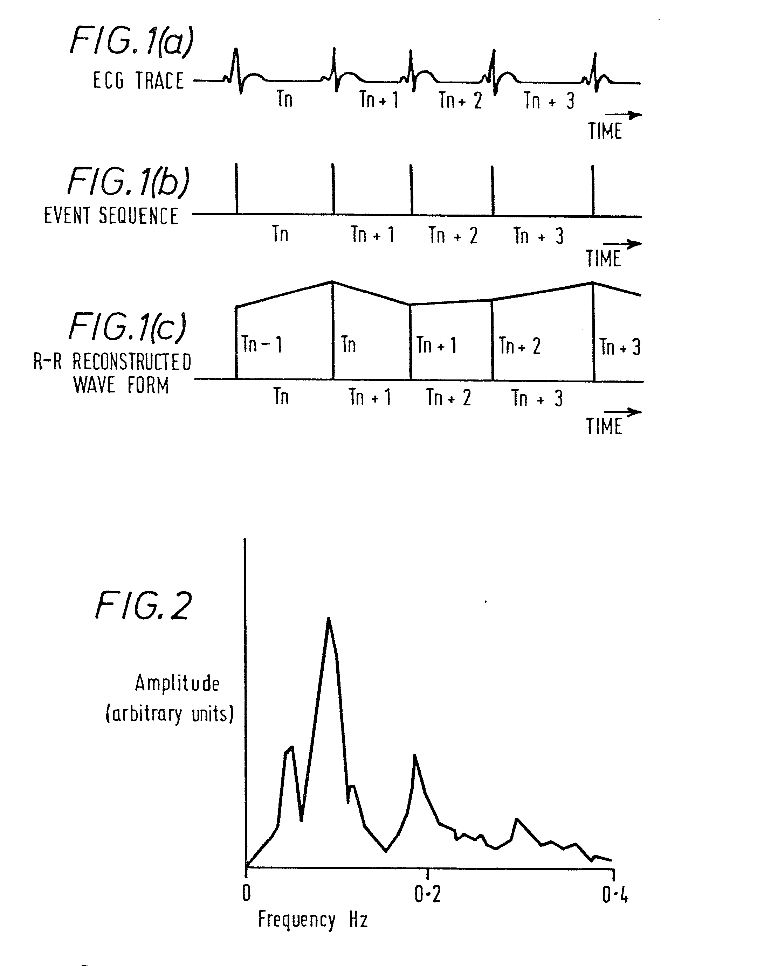

Figure 1 of the accompanying drawings illustrates the irregularity of the interval between heart beats of a human at rest. Figure 1A shows the E.C.G. trace, the time intervals between beats being expressed by $T_n$, $T_{n+1}$, $T_{n+2}$, etc. Figure 1B is similar to Figure 1A but with the individual beats reduced to a single line. The interbeat interval is usually referred to as the RR interval which is in fact the interval between one R wave and the next, the R wave referring to the largest peak in the electrocardiograph pattern. Figure 1C shows a wave form constructed from Figure 1B in which the amplitude of a particular line beat is proportional to the interval between that beat and the preceding beat. Such an interpolated wave form may be analysed using fast Fourier transform techniques to provide an analysis of the irregular heart beat intervals such as is shown in Figure 2 of the accompanying drawings.

Various methods for interpolating the heart beat signal intervals have been described by Luczak et al (Luczak H. and Laurig, W. (1973), An Analysis of Heart Rate Variability Ergonomics, 16 (1) pp 85 - 97) and Chess/(Chess, C.F., Tam, R.M.K. and Calaresu, F.R. (1975), Influence of Cardiac Neural Inputs on Rhythmic Variations of Heart Period in the Cat. Journal of Physiology, 28 (3), pp 775 - 780). The analysis of such an interpolated wave form is described by Galloway et al (Galloway, D.F. and Womack, B.F. (1969), An Application of Spectral Analysis

0019444

- 4 -

and Digital Filtering to the Study of Respiratory Sinus
Arrhythmia. Electronic Research Centre, University of Texas,
Austin Technology Report 71, pp 11 - 47). Alternatively, a
signal average or autocorrelator can be used for one signal to
give the spectrum. Similarly the cross-spectrum of two signals
can be found from the cross-correlation of the signals.
Other techniques that can be used are a digital statistical
signal analyser and tracking filters.

Referring again to the example of an analysed signal shown
in Figure 2, the first peak, which occurs in the region 0.03-0.05
corresponds
Hz,/to the body thermal control mechanism, the second peak at
0.09-0.11 Hz to the blood pressure control frequency referred to
as the Traube-Hering wave or the Mayer wave, and the third peak
to the respiration frequency in a subject at rest. However, there
are often several subsidiary peaks that can be attributed to
harmonics or irregular breathing which can occur during stress.

Embodiments of the present invention will now be described,
by way of examples only, and with reference to the remaining
Figures of the accompanying drawings.

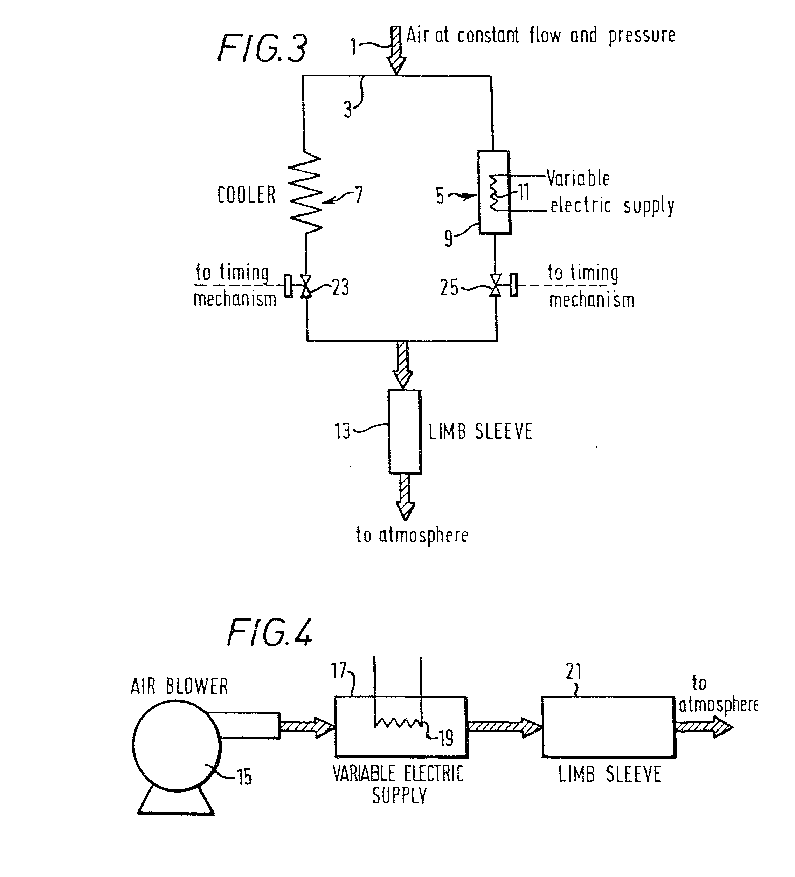

Referring to Figure 3 of the drawings, a first embodiment of
apparatus in accordance with the present invention includes means
for applying alternating hot and cold air to the limb of a
subject, these means being shown diagrammatically in Figure 3.
The equipment includes means (not shown) for supplying air at
constant pressure, flow and temperature, indicated by arrow 1 to
piping and valve system 3 where it is alternately heated and
cooled by switching through either the heating device 5 or the
cooling device 7. Heating device 5 comprises a chamber 9
containing an electrical heater 11. By controlling the power to
the heater the gas emerging from chamber 9 has a constant warm
temperature. The power to the heater may be controlled by means
of a variac by means of which the voltage may be adjusted
manually to give the desired air temperature or by using a
thyristor circuit and manually altering the bias of the
thyristor triggering unit thereby changing the power supply to the heater. The cooling device comprises a coil through which the gas is passed, the coil either being immersed in a constant temperature bath or, if the room temperature is low enough, merely left in the atmosphere.

The hot or cold air passes from piping and valve system 3 into a plastics sheath 13 which surrounds part of either a leg or arm of the patient. The air is allowed to escape from the opposite end of the plastics sheath into the atmosphere. Air is used as it does not contaminate the limb or the atmosphere but any other gas may also be used provided it does not interfere with the subject or contaminate the atmosphere. The gas may be recirculated if desired. Typically the temperature of the cold air is $18^{\circ}$ C and the temperature of the hot air $46^{\circ}$C.

In an alternative embodiment, as illustrated in Figure 4, air is supplied by air blower 15 to chamber 17 in which is contained a heater 19. The air blower may be an industrial hot air blower similar to a ladies hair dryer. This air blower 15 is operated continuously and hot or cold air is provided by switching on or off the heating element of the blower. The hot air temperature is controlled by measuring the air temperature and varying the voltage delivered to heater 19. This method can only be used where the room air temperature is sufficiently low. The hot or cold air is passed to sheath 21 surrounding the subject's limb.

The sheath can be made of any material which is suitable for restricting the air flow around the limb and compatible with the limb. Typically the sheath is formed from a plastics tube having a thickness of about 0.4 mm. The sheath may be provided with a funnel fitted inside one end to ensure a uniform air distribution across the end of the limb.

The piping and valve system 3 (Figure 3) can be made of, for instance, plastics or copper of sufficient diameter to give an air flow at the exit points of about 0.15 $1s^{-1}$.

Referring again to Figure 3, the valves 23 and 25 of piping and valve sytem 3 have associated with them timing devices (not

- 6 -

shown) which may be either mechanical or electronic. A typical electronic timing device includes a constant frequency oscillator of between 20 and 200 Hz which is divided down and drives a bistable device. The period over which hot or cold air is applied may be varied by varying the frequency of the oscillator and/or the many dividing circuits. The bistable device drives a relay which operates solenoid valves 23 and 25 and also sends a signal to the analyser device if required.

The electrical signals, in the form of an electrocardiogram or other signal generated by the body, are either coupled to a magnetic tape recorder or to a data processing unit, usually real time computer, via an interface which may process the signal before feeding the information to the computer.

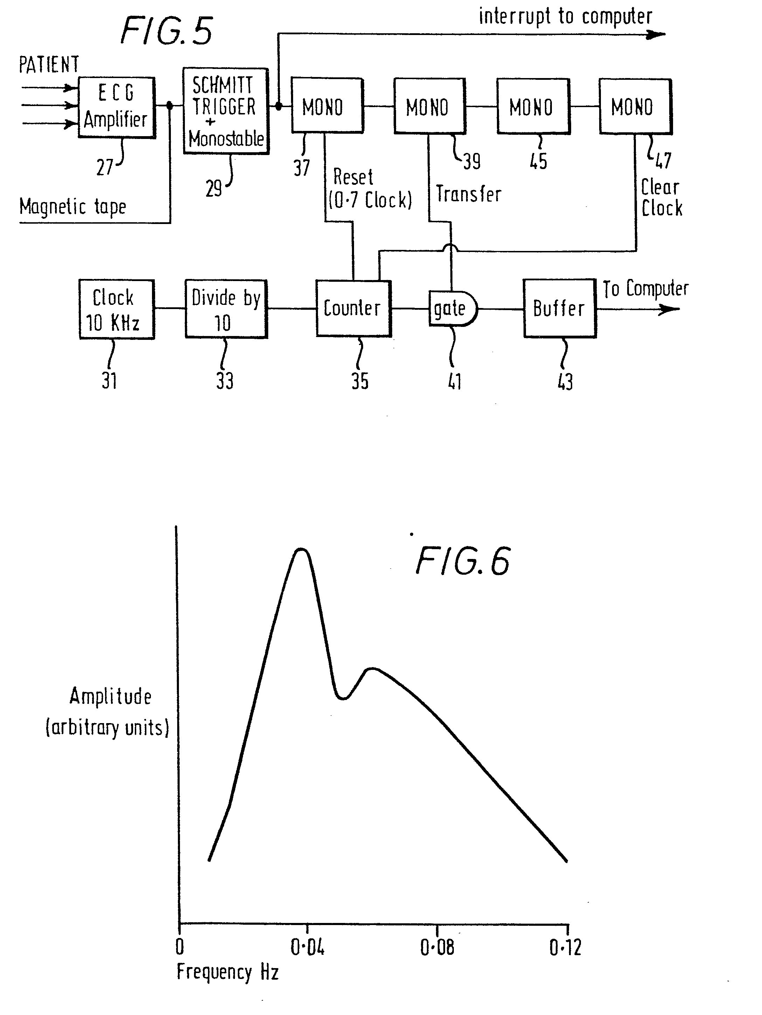

Figure 5 is a block diagram of an example of an arrangement for treating an E.C.G. signal. The E.C.G. signal is fed into a three input differential amplifier 27 which is specially developed for E.C.G. amplification and has a small gain of 15. The amplifier 27 has two stages which are optically coupled and its ouput is fed into another amplifier (not shown separately ) with an adjustable gain from 100 to 300. An offset facility is also provided so that the mean level of the output can be present. The signal amplitude at the output of this second amplifier is typically in the range 0.5 V to 1.5 V. This signal can be fed into an analogue magnetic tape recorder for storage and/or fed directly onto the rest of the circuit. The reduce base line drift, which occurs with some subjects, a differentiating circuit is sometimes used between the two amplifiers. The time constant of the differentiators can be switched in various ranges from 0.05 s to 1 s.

The E.C.G. signal from either the amplifier or the tape recorder is fed into a Schmitt trigger 29 which triggers at or near the peak of the R wave. This in turn sets off a series of monostables in sequence, the outputs of which generate signals to control the timing circuits. Another output from the Scmitt trigger is fed to the digital computer to indicate that the R wave has just appeared. The timing circuit is illustrated in the lower line of Figure 5. A 10 KHz clock 31 is divided down to 1 KHz by divider 33 and then into a counter 35 which will count the time intervals required. The first monostable 37 stops the counter, the second monostable 39 opens the gate 41 so that the number in the counter is transferred into a buffer store 43. The third monostable 45 introduces a delay and the fourth monostable 47 resets the counter so it will time the next interval. The operation of all four monostables takes place within half a period of the clock pulse. The contents of the buffer store can be read at any time in the interval between one operation of the Schmitt trigger 29 and the next. It is usually read some two or three ms after the computer has received the interrupt from the Scmitt trigger 29. The register outputs are in binary coded decimal and are fed to the digital inputs of the computer.

The RR interval time is transferred to the computer and a wave form reconstructed as shown in Figure 1C. This wave form is then sampled every 400 ms and the results fed into a fast Fourier analogue programme. A typical set of results from this programme is illustrated in Figure 2:

An indication of the degree of entrainment can be obtained by using a filter at the forcing frequency and measuring the amount of the signal which comes through at that frequency.

In operation the alternation of hot and cold air flow to the patient's limb is set for periods between 4 s and 120 s and the subject is allowed to accommodate to the temperature at variation for a short period (about 5 minutes) and then the body parameters (for instance, the heart beat) recorded or analysed direct for a further 5 minutes. This procedure is then repeated at different frequencies.

For the best results the subject should have a core temperature of $37^{\circ}C$, peripheral temperature of $34^{\circ}C$ and a pulse rate of less than 90 beats per minute, preferably below 70 beats per minute and in sinus rhythm.

- 8 -

It is found that for a period time of from 8 s to 80 s the body of an adult can follow the periodic temperature variations, that is to say, this is the range of frequencies over which entrainment takes place.

Normal human subjects have the greatest entrainment with period times of approximately 30 seconds. No entrainment at all occurs with a period time of greater than 80 s or less than 8 s. At rest the normal body/control mechanism oscillates at 0.03 Hz so it is not surprising that the best entrainment occurs close to this frequency. This entrainment is shown clearly in the autospectrum of the cardiac rate and peripheral blood flow signals and in the cross-spectrum of the two. It is not apparent in the respiration signal.

Figure 6 is a graph illustrating how the response of the thermal control mechanism varies with the forcing frequency. From this graph and capture ratio can be defined where the bandwidth is defined by the usual techniques. The average capture ratio for a small sample of normal adults was 24 s and for a subject with a slow heart rate was 21 s, and accordingly the heart rate is not important when defining bandwidth.

Apparatus in accordance with the present invention may be used in the following applications :-

(1) To test the integration of the autonomic nervous system in applications such as:-

(a) early detection of autonomic disfunction in diabetics by periodic screening;

(b) differential diagnosis of postural hypotension; and

(c) clinical estimation of dose response of anti-hypotensive agents, particularly ganglion blocking agents.

(2) Testing normal regularity responses of temperature control;

(3) In intensive care situations to test the adequacy of blood volume replacement and cardiact output;

(4) In health clinics and physical fitness training schemes to develop the improvements in the thermal control and work rate relationships;

(5) In investigations of congenital heart disease, central shunts, notably strial and ventricular septal defects, in patients with cyanotic diseases and pulmonary oligaemia as well as investigations of acquired heart disease including patients with pulmonary plethora or pulmonary venous hypertension;

(6) In physcological investigations including a determination of attention span and concentration;

(7) In investigations of peripheral vascular disease particularly microangiopathy (i.e. reynaud's phenomena) and attesting of the many drugs concerned with the peripheral blood flow.

- 1 -

CLAIMS:

1.    Apparatus for determining the response of an animal body to periodic application of an external stimulus, characterised in that the apparatus comprises means for applying alternating hot and cold gas to at least a part of the animal body, means for presenting a periodic signal generated by the animal body, and detected during the application of said hot and cold gas, in a form suitable for assessing the response of said signal to said application of alternating hot and cold air.

2.    Apparatus according to claim 1 characterised in that the apparatus further includes means for measuring said period signal.

3.    Apparatus according to claim 1 characterised in that the means for presenting the periodic signal include means for accurately recording the intervals between characteristic repeat portions of the signal and means for analysing the results obtained to isolate the contribution to the signal provided by the thermoregulatory control mechanism.

4.    Apparatus according to claim 3 characterised in that the analysis means include means for analysing by Fourier transform techniques.

5.    Apparatus according to any of the preceding claims characterised in that the gas is air and it is applied to the limb of the animal.

6.    A method for determining the response of an animal body to periodic application of an external stimulus characterised in that the method comprises applying alternating hot and cold gas to at least a part of the animal body, detecting a periodic signal generated by the animal body during the application of

- 2 -

said alternating hot and cold air, and presenting the
detected periodic signal in a form suitable for
assessment of the response of said signal to said
application of alternating hot and cold air.

FIG.1(a)
ECG TRACE

FIG.1(b)
EVENT SEQUENCE

FIG.1(c)
R-R RECONSTRUCTED
WAVE FORM

FIG.2

Amplitude
(arbitrary units)

Frequency Hz

FIG.3

Air at constant flow and pressure

1

3

COOLER 7

5 11 Variable electric supply

9

to timing mechanism 23

25 to timing mechanism

13 LIMB SLEEVE

to atmosphere

FIG.4

AIR BLOWER

17

19

15

VARIABLE ELECTRIC SUPPLY

21

LIMB SLEEVE

to atmosphere

FIG.5

interrupt to computer

PATIENT

ECG Amplifier
27

SCHMITT TRIGGER + Monostable
29

MONO 37

MONO 39

MONO 45

MONO 47

Magnetic tape

Reset (0·7 Clock)

Transfer

Clear Clock

Clock 10 KHz
31

Divide by 10
33

Counter
35

gate
41

Buffer
43

To Computer

FIG.6

Amplitude (arbitrary units)

Frequency Hz

0        0·04        0·08        0·12

## European Patent Office

## EUROPEAN SEARCH REPORT

| | DOCUMENTS CONSIDERED TO BE RELEVANT | | CLASSIFICATION OF THE APPLICATION (Int. Cl. ¹) |
|---|---|---|---|
| Category | Citation of document with indication, where appropriate, of relevant passages | Relevant to claim | |
| | US - A - 3 563 231 (B.A. DUCOTE & L.V. NICASTRO/TRACOR, INC.) <br><br> * Abstract; column 1, lines 29-71; column 2, line 47 - column 3, line 7; column 4, line 64 - column 5, line 41; figures * <br><br> -- | 1-3,6 | A 61 B 5/00 <br> A 61 F 7/00 <br> A 61 H 33/06 <br> A 61 B 5/04 |
| | US - A - 3 794 017 (G.H. SERVOS) <br><br> * Abstract; column 1, lines 42-65; column 2, line 19 - column 4, line 44; column 5, line 33 - column 6, line 6; figures 1-3 * <br><br> -- | 1-3,6 | |
| | US - A - 3 000 271 (S.C. HARVEY et al.) <br><br> * Column 1, lines 24-48; column 2, line 54 - column 3, line 50; column 4, line 19 - column 5, line 14; column 5, lines 15-24; figures 1-11 * <br><br> -- | 1-3,5, 6 | TECHNICAL FIELDS SEARCHED (Int.Cl. ³) <br><br> A 61 B 5/00 <br> 5/04 <br> A 61 D 7/00 <br> A 61 F 7/00 <br> 7/12 <br> A 61 H 33/06 <br> A 61 M 19/00 |
| | US - A - 3 776 241 (J.H. MAGILTON & C.S. SWIFT/ IOWA STATE UNIVERSITY RESEARCH FOUNDATION) <br><br> * Abstract; column 1, lines 5-24; column 2, lines 46-65; column 3, line 55 - column 4, line 34; column 8, lines 50-64; column 9, lines 27-62; column 13, line 31 - column 14, line 61; figures 1-4 * <br><br> -- | 1-3,6 | CATEGORY OF CITED DOCUMENTS <br><br> X: particularly relevant <br> A: technological background <br> O: non-written disclosure <br> P: intermediate document <br> T: theory or principle underlying the invention <br> E: conflicting application <br> D: document cited in the application <br> L: citation for other reasons |
| | GB - A - 485 811 (B. HILL) <br><br> * Page 1, lines 9-42; page 2, lines 27-115; page 3, lines 31-63; page 4, lines 20-121; | 1,5 | &: member of the same patent family. <br> corresponding document |

X | The present search report has been drawn up for all claims | ./.

| Place of search | Date of completion of the search | Examiner |
|---|---|---|
| The Hague | 13-08-1980 | RIEB |

EPO Form 1503.1  06.78

0019444

European Patent
Office

**EUROPEAN SEARCH REPORT**

Application number

EF 8J 3J 15-7

- 2 -

| | DOCUMENTS CONSIDERED TO BE RELEVANT | | CLASSIFICATION OF THE APPLICATION (Int. Cl. 3) |
|---|---|---|---|
| Category | Citation of document with indication, where appropriate, of relevant passages | Relevant to claim | |
| | figures 1-16 *<br><br>--<br><br>US - A - 2 832 336 (C.D. DAVIS & W.M. YOCUM)<br><br>  * Column 1, line 29 - column 2, line 13; column 2, line 28 - column 3, line 38; figures 1-3 *<br><br>-- | 1,5,6 | |
| | ELECTRONICS AND POWER, vol. 22, no. 8, August 1976, pages 510-514<br>Hitchin, G.B.<br>K.R. GODFREY et al.: "Computer analysis of brainwaves"<br><br>  * Page 510, abstract; pages 513-514, the section "Computer analysis of ongoing activity" *<br><br>-- | 2,4 | TECHNICAL FIELDS SEARCHED (Int. Cl. 3) |
| | US - A - 3 404 678 (M. VON ARDENNE)<br><br>  * Abstract; column 2, lines 18-45; column 4, lines 4-23; figures *<br><br>-- | 1,2 | |
| | US - A - 4 044 772 (B. SCHLOSS)<br><br>  * Abstract; column 3, lines 39-57; column 6, lines 29-49; column 8, line 47 - column 9, line 12; figures 1,3 *<br><br>---- | 1,2 | |